# EUROPEAN PATENT SPECIFICATION

(11) **EP 3 055 011 B1**
(45) Date of publication and mention of the grant of the patent: **31.07.2019**
(21) Application number: 14798709.3
(22) Date of filing: 06.10.2014
(51) Int. Cl.: A61M 16/04, A61M 16/10, A61M 16/20

(54) **BREATHING PROTECTOR**
ATEMSCHUTZ
PROTECTEUR RESPIRATOIRE

(30) Priority: 09.10.2013 SE 1351197
(43) Date of publication of application: 17.08.2016
(73) Proprietor: Atos Medical AB, 242 22 Hörby (SE)
(72) Inventor: HESSELMAR, Bill, S-472 94 Svanesund (SE); PERSSON, Jan-Ove, S-243 31 Höör (SE)
(74) Representative: Ström & Gulliksson AB
(86) International application number: PCT/EP2014/071314
(87) International publication number: WO 2015/052121

(56) References cited:
- US-A1- 2002 156 527
- US-A1- 2004 089 291
- US-A1- 2004 123 868
- US-A1- 2011 120 473

## Description

### Field of the Invention

This invention pertains in general to the field of breathing protectors, such as tracheostoma valves, and more specifically to a tracheostoma valve, comprising a main valve, being in an open position during inhalation and in a closed position during normal vegetative exhalation, to permit speech, and a relief valve, being in a closed position during normal vegetative exhalation and transiting into an open position when the pressure in the trachea and thus the tracheostoma valve exceeds a threshold value corresponding to a discomforting high pressure in the trachea.

### Background of the Invention

Individuals with tracheotomy tubes often have difficulty with speech, which is normally produced by airflow past the vocal cords on expiration. If a tracheotomy tube is present, air on expiration is mainly expelled through the tracheotomy tube rather than through the vocal cords. To overcome this problem, a one-way tracheotomy valve can be placed over the entrance to the tracheotomy tube. This allows air to be inhaled through the valve and into the tracheotomy tube, but does not allow exhaled air to escape back through the tracheotomy tube and out the valve. Instead, the air is forced around the tracheotomy tube, and escapes through the vocal cords, permitting speech. The corresponding increase in subglottic pressure during exhalation also facilitates swallowing and cough production.

However, some people with a tracheotomy tube, particularly children, cannot tolerate a speaking valve because their airway is too obstructed by the tube to allow full and comfortable clearance of air inspired through the one-way valve. This may be caused by an upper tracheal or subglottic stenosis, suprastomal collapse, or too large a tracheotomy tube. These conditions limit the escape of inhaled air, and may result in a feeling of suffocation and raised intrathoracic pressure on expiration. This may cause the speaking valve to be displaced, or may result in chronically raised intrathoracic pressure which, in turn, may compromise venous blood return to the heart and predispose one to pulmonary hypertension.

US 2004/0123868 discloses a tracheotomy valve unit for use with a tracheotomy tube inserted into a patient's trachea. The tracheotomy valve unit comprises a first valve that permits airflow to the tube when the patient inhales and blocks airflow from the tube when the patient exhales thereby permitting speech, and a second valve that permits airflow from the tube back out the valve unit when the intrathoracic pressure during expiration is high. The second valve is a slit in a silicone diaphragm or silicone umbrella valve, located in the central rivet. Thus, the resistance of the second valve has a very low consistency, i.e. a low repeatability, being dependent on moisture in slit or valve interface and temperature, since wear and changes in temperature of the silicone diaphragm or umbrella will change its closing and resisting characteristics. Additionally, the second valve cannot adapt to rapid increases in pressure, and will thus in some instances take an uncomfortable long time to neutralize the too high intrathoracic pressure. Also, the second valve is facing axially, thus being prone to be obstructed by clothes etc. Still further, the second valve is very difficult to control with regard to cut off pressure, since the active surface area of the second valve is low.

US 4,582,058 discloses a tracheostoma valve assembly containing a spring-biased main valve that remains open during normal breathing and closes during normal air flow associated with speech. The valve assembly includes a separate external relief valve that is closed during normal breathing and speaking and opens to release air pressure resulting from a substantially increased air pressure within the valve assembly, such as due to a cough. The relief valve automatically closes when the air pressure is reduced in the valve assembly. The release valve is directed radially in the assembly, and comprises housing through holes and a rubber band. Thus, the resistance of the release valve has a very low consistency, being dependent on moisture in hole/rubber band interface and temperature. Additionally, the second valve cannot adapt to rapid increases in pressure, and will thus in some instances take an uncomfortable long time to neutralize the too high intrathoracic pressure. Still further, due to the release valve being made of a rubber band, the repeatability of the release valve is low, since rubber will change rapidly in characteristics due to wear and ambient conditions. Hence, the release valve cut off is difficult to control.

Thus, there is a need for a breathing protector unit that allows full and comfortable clearance of inspired air and prevents the build up of excess intrathoracic pressure during expiration, while maintaining sufficient pressure to allow the user to generate speech, said breathing protector providing an adjustable intrathoracic pressure release, such that pressure release function of the breathing protector is improved with regard to repeatability and is highly independent of moisture and temperature as well as rapidness of building up the intrathoracic pressure.

The following documents also disclose tracheostoma valves, US 2002/0156527, US 2011/0120473 and US 2004/0089291.

### Summary of the Invention

The present invention is defined in independent claim 1. the present invention preferably seeks to mitigate, alleviate or eliminate one or more of the above-identified deficiencies in the art and disadvantages singly or in any combination and solves at least the above mentioned problems by providing a breathing protector to be fitted in a tracheostoma, comprising; a housing with a distal part and a proximal part, a first opening at a distal end of the distal part, and a second opening at the proximal end of the breathing protector; a valve flap arranged at the first opening to closingly engage with a valve seat circumferentially of the first opening, said valve flap and valve seat creating a main valve being in an open position during inhalation and in a closed position during normal vegetative exhalation through the breathing protector; a spring member spring biasing the distal part in relation to the proximal part, to create a relief valve, being in a closed position during normal vegetative exhalation and transiting into an open position when the pressure in the trachea and thus the breathing protector exceeds a threshold value corresponding to a discomforting high pressure in the trachea.

Advantageous embodiments will be apparent from the for example the appended claims.

### Brief Description of the Drawings

These and other aspects, features and advantages of which the invention is capable of will be apparent and elucidated from the following description of embodiments of the present invention, reference being made to the accompanying drawings, in which
Fig. 1 is a perspective view of a tracheostoma device according to an embodiment of the present disclosure;
Fig. 2 is a cross sectional view of a tracheostoma device according to an embodiment of the present disclosure with a closed release valve;
Fig. 3 is a cross sectional view of a tracheostoma device according to an embodiment of the present disclosure with an open release valve;
Fig. 4 is an exploded perspective and cross sectional view of a tracheostoma device according to an embodiment of the present invention; and
Fig. 5 is an exploded and perspective view of a tracheostoma device according to an embodiment of the present invention.

### Description of embodiments

The following description focuses on an embodiment of the present invention applicable to a trachestoma device, and particularly to a tracheostoma device with a valve member for allowing vegetative inhalation there through.

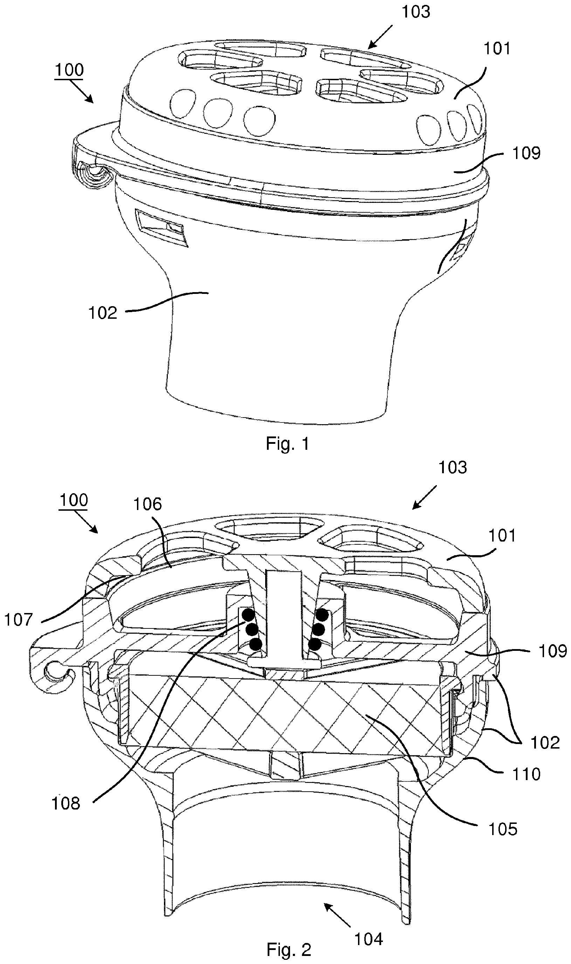

A breathing protector 100, such as a tracheostoma valve, to be fitted in a tracheostoma is illustrated in Figs. 1 and 2. The breathing protector 100 comprises a housing 100. The housing 100 comprises a distal part 101 and a proximal part 102. At a distal end of the distal part 101 a plurality of openings 103 are evenly distributed. The openings 103 are evenly distributed to distribute inhaled air over a large width of the breathing protector 100, and to ensure that the risk of occluding the breathing protector 100 is minimized. It is also possible, even though not preferred, to have only one opening 103 at the distal end of the distal part 101.

At a proximal end of the breathing protector 100, such as a proximal end of the proximal part 102, a second opening 104 is provided. A moisture and heat exchanging filter 105 is arranged within the breathing protector 100, such that inhaled air will pass the moisture and heat exchanging filter 105. The moisture and heat exchanging filter 105 may be arranged between grids on the distal part 101 and the proximal part 102, respectively, such that its position in the breathing protector 100 may be relatively fixed. A valve flap 106 is arranged proximally of the plurality of openings 103, such that the valve flap 106 will closingly engage with the plurality of openings 103 during exhalation, thus preventing exhalation through the breathing protector 100 exiting the openings 103. This may be realized by suspending the valve flap 106 centrally and proximally of the distal end wall of the distal part 101. This may be accomplished by riveting the valve flap 106 to the distal end wall of the distal part 101. The circumferencial edge of the openings 103 may thus constitute a valve seat 107, or a plurality of valve seats 107, depending on the number of openings 103.

The valve seat(s) 107 and the valve flap 106 together create a main valve being in an open position during inhalation and in a closed position during normal vegetative exhalation through the breathing protector 100. The main valve will open upon inhalation through pulling the valve flap 106 away from interaction with the valve seat 107, by means of the under pressure in the lungs. During exhalation on the other hand, the valve flap 106 will be pushed against the valve seat 107 to close the openings 103 by means of the over pressure in the lungs, and the air will instead be expelled through trachea or a fistula in the oesophagal wall.

A spring member 108 is arranged between the distal part 101 and the proximal part 102 to create a relief valve, being in a closed position during normal vegetative exhalation and transiting into an open position when the pressure in the trachea and thus the breathing protector 100 exceeds a threshold value corresponding to a discomforting high pressure in the trachea. The distal part 101 may be suspended in relation to the proximal part 102 via the spring member 108. In this case, the distal part 101 may be suspended centrally of the breathing protector 100 by the spring member 108, which in turn is connected to the proximal part 102. The spring member 108 may be spring coil of a suitable metal or plastic, and its spring resistance may be adapted after the precise and wished for resisting force to create a relief valve, in accordance with above, i.e. being in a closed position during normal vegetative exhalation and transiting into an open position when the pressure in the trachea and thus the breathing protector 100 exceeds a threshold value corresponding to a discomforting high pressure in the trachea. The threshold value may preferably be set in the interval of from 4 to 12 cm H₂O. When the breathing protector 100 is provided with a moisture and heat exchanging filter 105, the spring member 108 may be of plastic. This may be preferable for example when the moisture and heat exchanging filter 105 has been provided with a suitable salt, to decrease corrosion of the spring member 108. The spring member 108 may have a conical shape, to minimize the proximodistal extension of the spring member 108, while simultaneously providing for a suitable number of coil revolutions for increasing the possibility to regulate and control the resisting force of the spring member 108 to match and keep the discomfort threshold value.

The interaction between the distal part 101 and the proximal part 102, in closed position of the relief valve, may be at the periphery of the distal part 101 and the proximal part 102, respectively, due to the spring member suspension centrally of the breathing protector 100, thus allowing for a large cross sectional pressure area. This in turn gives an improved control and regulation of the relief valve threshold value. Additionally, all exhaled air will pass through the breathing protector 100 and the relief valve in substantially normal exhalation time. This means that the breathing protector 100 also can be used with the presence of relatively serious constrictions of the respiratory passages, such as in connection to cannula.

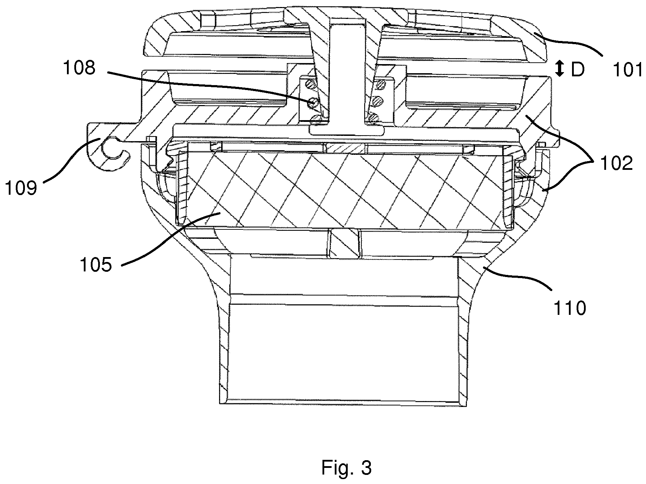

In Fig. 3 the breathing protector is disclosed in an open position of the relief valve, where it can be visualized how the distal part 101 and the proximal part 102 are separated with a distance D upon application of a pressure in the breathing protector 100 exceeding the comfort threshold value.

The proximal part 102 may comprise an upper proximal part 109 and a lower proximal part 110, to facilitate assembly of the heat and moisture exchanging filter 105 in the proximal part 102. The upper proximal part 109 and the lower proximal part 110 may be provided with grids, respectively, between which the heat and moisture exchanging filter 105 may be held in place.

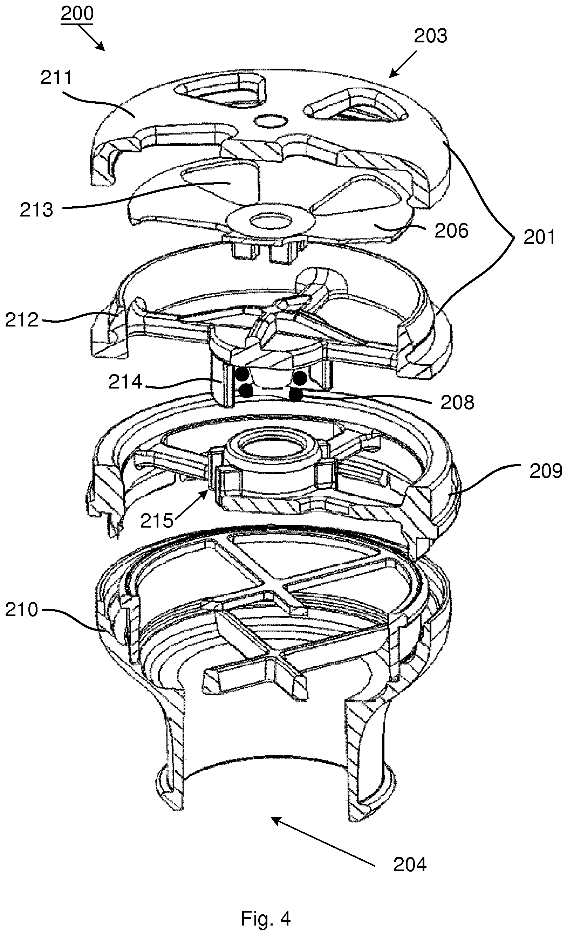

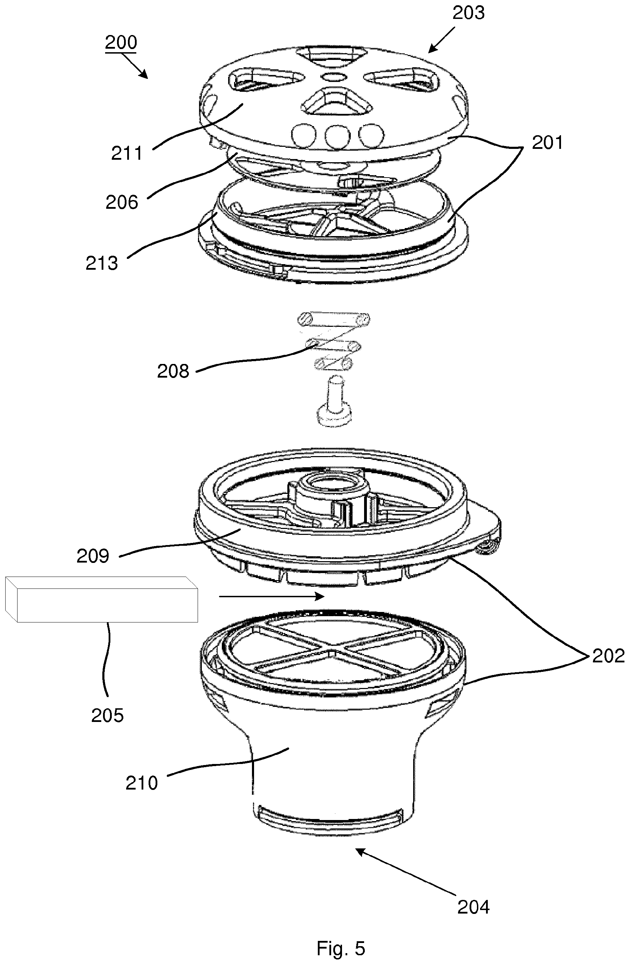

In another embodiment, in accordance with Figs. 4 and 5, a breathing protector 200 is disclosed, said breathing protector 200 comprising a distal part 201, said distal part 201 in turn comprising a lid 211 and a distal part support structure 212. The lid 211 is rotatable in the transversal plane of the breathing protector 200 in relation to the support structure 211. A valve disc 206 is arranged on the distal part support structure 212, such that the lid 211 is rotatable in the transversal plane of the breathing protector 200 in relation to the valve disc 206. The valve flap disc is provided with apertures 213, such that distal openings 201 will be superimposed the apertures 213, such that a free exhalation passage is obtained through the first opening 203 in a free exhalation position of the breathing protector 200. When rotating the lid 211 into another position in relation to the valve disc 206, the distal openings 203 of the lid 211 will be superimposed continuous parts of the valve disc 206, such that exhalation through the distal openings 203 is prevented in a closed exhalation position of the breathing protector 200.

The valve disc 206 may in one embodiment be arranged on the support structure 212. The support structure 212 is provided with at least one locking tap 213, such as two locking taps 214, extending proximally from said support structure 212 to slide engagingly in the proximodistal direction in a locking groove 215 arranged in the proximodistal direction on the proximal part 202, to prevent rotation of the valve disc 206 in the transversal plane of the breathing protector 200. The locking taps 214 and the locking grooves 215 may be suspended centrally of the breathing protector 200.

In accordance with the embodiment according to Figs. 1 to 3, the breathing protector 200 comprises a housing 200. The housing 200 comprises the distal part 201 and a proximal part 202. At the distal end of the distal part 201 a plurality of openings 203 are evenly distributed. The openings 203 are evenly distributed to distribute inhaled air over a large width of the breathing protector 200, and to ensure that the risk of occluding the breathing protector 200 is minimized. It is also possible, even though not preferred, to have only one opening 203 at the distal end of the distal part 201. Then the valve disc 206 only need to have one aperture 213, which may be rotated to be superimposed the opening 203.

At a proximal end of the breathing protector 200, such as a proximal end of the proximal part 202, a second opening 204 is provided. A moisture and heat exchanging filter 205 is arranged within the breathing protector 200, such that inhaled air will pass the moisture and heat exchanging filter 205. The moisture and heat exchanging filter 205 may be arranged between grids on the distal part 201 and the proximal part 202, respectively, such that its position in the breathing protector 200 may be relatively fixed. The valve disc 206 is arranged proximally of the plurality of openings 203, as disclosed and indicated above, such that the valve disc 206 may closingly engage with the plurality of openings 203 during exhalation, in the closed exhalation position, thus preventing exhalation through the breathing protector 200 exiting the openings 203. This may be realized by suspending the valve disc 206 centrally and proximally of the distal end wall of the distal part 201. This may be accomplished by riveting the valve disc 206 to the distal end wall of the distal part 201, such that the valve disc 206 still can be rotated in relation to the lid 211. The circumferencial edge of the openings 203 may thus constitute a valve seat 207, or a plurality of valve seats 207, depending on the number of openings 203.

The valve seat(s) 207 and the valve disc 206 together create a main valve being in an open position during inhalation and in a closed position during normal vegetative exhalation through the breathing protector 200. The main valve will open upon inhalation through pulling the valve disc 206 away from interaction with the valve seat 207, by means of the under pressure in the lungs. During exhalation on the other hand, the valve flap 206 will be pushed against the valve seat 207 to close the openings 203 by means of the over pressure in the lungs, and the air will instead be expelled through trachea or a fistula in the oesophagal wall, when the lid 211 has been rotated in the transversal plane in relation to the valve disc 206 into the closed exhalation position.

A spring member 208 is arranged between the distal part 201 and the proximal part 202 to create a relief valve, being in a closed position during normal vegetative exhalation and transiting into an open position when the pressure in the trachea and thus the breathing protector 200 exceeds a threshold value corresponding to a discomforting high pressure in the trachea. The distal part 201 may be suspended in relation to the proximal part 202 via the spring member 208. In this case, the distal part 201 may be suspended centrally of the breathing protector 200 by the spring member 208, which in turn is connected to the proximal part 202. The spring member 208 may be spring coil of a suitable metal, and its spring resistance may be adapted after the precise and wished for resisting force to create a relief valve, in accordance with above, i.e. being in a closed position during normal vegetative exhalation and transiting into an open position when the pressure in the trachea and thus the breathing protector 200 exceeds a threshold value corresponding to a discomforting high pressure in the trachea. The spring member 208 may have a conical shape, to minimize the proximodistal extension of the spring member 208, while simultaneously providing for a suitable number of coil revolutions for increasing the possibility to regulate and control the resisting force of the spring member 208 to match and keep the discomfort threshold value.

The interaction between the distal part 201 and the proximal part 202, in closed position of the relief valve, may be at the periphery of the distal part 201 and the proximal part 202, respectively, due to the spring member suspension centrally of the breathing protector 200, thus allowing for a large cross sectional pressure area. This in turn gives an improved control and regulation of the relief valve threshold value.

The proximal part 202 may comprise an upper proximal part 209 and a lower proximal part 210, to facilitate assembly of the heat and moisture exchanging filter 205 in the proximal part 202. The upper proximal part 209 and the lower proximal part 210 may be provided with grids, respectively, between which the heat and moisture exchanging filter 205 may be held in place.

In the claims, the term "comprises/comprising" does not exclude the presence of other elements or steps. Furthermore, although individually listed, a plurality of means, elements or method steps may be implemented by e.g. a single unit or processor. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality. Reference signs in the claims are provided merely as a clarifying example and shall not be construed as limiting the scope of the claims in any way.

## Claims

1. A breathing protector (200) to be fitted in a tracheostoma, comprising; a housing (200) with a distal part (201) and a proximal part (202), a first opening (203) at a distal end of the distal part (201), and a second opening (204) at the proximal end of the breathing protector (200); a valve flap (206) arranged at the first opening (201) to closingly engage with a valve seat (207) circumferentially of the first opening (201), said valve flap (206) and valve seat (207) creating a main valve being in an open position during inhalation and in a closed position during normal vegetative exhalation through the breathing protector (200); a spring member (208) spring biasing the distal part (101, 201) in relation to the proximal part (202), to create a relief valve, being in a closed position during normal vegetative exhalation and transiting into an open position when the pressure in the trachea and thus the breathing protector (200) exceeds a threshold value corresponding to a discomforting high pressure in the trachea; wherein the distal part (201) comprises a lid (211), said lid (211) comprising the first opening (203) and being rotatable in the transversal plane of the breathing protector (200) in relation to the valve flap (206), such that a free exhalation passage is obtained through the first opening (203) in a free exhalation position.

2. The breathing protector according to claim 1, wherein the distal part (201) is suspended in relation to the proximal part (202) via the spring member (208) centrally of the breathing protector (200).

3. The breathing protector according to claim 1 or 2, wherein the proximal part (202) comprises an upper proximal part (209) mounted on a lower proximal part (210), and a moisture and heat exchanging filter (205) is arranged between the upper proximal part (209) and the lower proximal part (210).

4. The breathing protector (200) according to any of the preceding claims, further comprising a moisture and heat exchanging filter (205) received in said housing (200).

5. The breathing protector (200) according to any of the preceding claims, wherein the valve flap (206) is a valve disc with an aperture (213), said aperture being superimposed said first opening (201) in said free exhalation position.

6. The breathing protector (200) according to any of the preceding claims, wherein the valve flap (206) is arranged on a distal part support structure (212) having at least one locking tap (214) extending proximally from said support structure (212) to slide engagingly in the proximo distal direction in a locking groove (215) arranged in the proximodistal direction on the proximal part (202), to prevent rotation of the valve flap (206) in the transversal plane of the breathing protector (200).

7. The breathing protector (200) according to claim 6, wherein the locking tap (214) and the locking groove (215) are suspended centrally of the breathing protector (200).

8. The breathing protector (200) according to any of the preceding claims, wherein the distal part (201) comprises several first openings (203).

9. The breathing protector (200) according to claim 8, wherein the several first openings (203) are evenly distributed on the distal end surface of the distal part (201).

10. The breathing protector (200) according to claim 9, wherein the valve flap (206) is a valve disc with several apertures (213), said apertures being superimposed said several first openings (201) in said free exhalation position.

## Patentansprüche

1. Atemschutzvorrichtung (200) zum Einfügen in eine Tracheostoma, umfassend;
ein Gehäuse (200) mit einem distalen Teil (201) und einem proximalen Teil (202), einer ersten Öffnung (203) an einem distalen Ende des distalen Teils (201) und einer zweiten Öffnung (204) am proximalen Ende der Atemschutzvorrichtung (200);
eine Ventilklappe (206), die an der ersten Öffnung (201) angeordnet ist, um in einen Ventilsitz (207) in Umfangsrichtung der ersten Öffnung (201) schließend einzugreifen, wobei Ventilklappe (206) und Ventilsitz (207) ein Hauptventil bilden in einer offenen Position während des Einatmens und in einer geschlossenen Position während des normalen vegetativen Ausatmens durch die Atemschutzvorrichtung (200);
ein Federelement (208), das den distalen Teil (101, 201) in Bezug auf den proximalen Teil (202) federnd vorspannt, um ein Überdruckventil zu bilden, das sich während des normalen vegetativen Ausatmens in einer geschlossenen Position befindet und in eine offene Position übergeht, wenn der Druck in der Luftröhre und damit in der Atemschutzvorrichtung (200) einen Schwellenwert überschreitet, der einem unangenehmen hohen Druck in der Luftröhre entspricht;
wobei der distale Teil (201) einen Deckel (211) aufweist, wobei der Deckel (211) die erste Öffnung (203) umfasst und in der Transversalebene der Atemschutzvorrichtung (200) in Bezug auf die Ventilklappe (206) rotierbar ist, so dass ein freier Ausatmungsdurchgang durch die erste Öffnung (203) in einer freien Ausatmungsposition erhalten wird.

2. Atemschutzvorrichtung nach Anspruch 1, wobei der distale Teil (201) in Bezug auf den proximalen Teil (202) über das Federelement (208) zentral von der Atemschutzvorrichtung (200) aufgehängt ist.

3. Atemschutzvorrichtung nach Anspruch 1 oder 2, wobei der proximale
Teil (202) einen oberen proximalen Teil (209) umfasst, der an einem unteren proximalen Teil (210) angebracht ist, und ein Feuchtigkeits- und Wärmeaustauschfilter (205) zwischen dem oberen proximalen Teil (209) und dem unteren Teil (210) angeordnet ist.

4. Atemschutzvorrichtung (200) nach einem der vorhergehenden Ansprüche, ferner umfassend ein Feuchtigkeits- und Wärmeaustauschfilter (205), der in dem Gehäuse (200) aufgenommen ist.

5. Atemschutzvorrichtung (200) nach einem der vorhergehenden Ansprüche, wobei die Ventilklappe (206) eine Ventilscheibe mit einer Öffnung (213) ist, wobei die Öffnung der ersten Öffnung (201) in der freien Ausatmungsposition überlagert ist.

6. Atemschutzvorrichtung (200) nach einem der vorhergehenden Ansprüche, wobei die Ventilklappe (206) an einer distalen Teilstützstruktur (212) angeordnet ist, die mindestens einen Verriegelungshahn (214) aufweist, der sich proximal von der Stützstruktur (212) erstreckt, um in einer proximodistalen Richtung in einer Rastnut (215), die in der proximodistalen Richtung angeordnet ist, einrastend zu gleiten, um eine Verdrehung der Ventilklappe (206) in der Transversalebene der Atemschutzvorrichtung (200) zu verhindern.

7. Atemschutzvorrichtung (200) nach Anspruch 6, wobei der Verriegelungshahn (214) und die Verriegelungsnut (215) mittig an der Atemschutzvorrichtung (200) aufgehängt sind.

8. Atemschutzvorrichtung (200) nach einem der vorhergehenden Ansprüche, wobei der distale Teil (201) mehrere erste Öffnungen (203) aufweist.

9. Atemschutzvorrichtung (200) nach Anspruch 8, wobei die mehreren ersten Öffnungen (203) gleichmäßig auf der distalen Endfläche des distalen Teils (201) verteilt sind.

10. Atemschutzvorrichtung (200) nach Anspruch 9, wobei die Ventilklappe (206) eine Ventilscheibe mit mehreren Öffnungen (213) ist, wobei die Öffnungen in der freien Ausatmungsposition den mehreren ersten Öffnungen (201) überlagert sind.

## Revendications

1. Protecteur respiratoire (200) à ajuster dans un trachéostome, comprenant ; un logement (200) avec une partie distale (201) et une partie proximale (202), une première ouverture (203) à une extrémité distale de la partie distale (201), et une seconde ouverture (204) à l'extrémité proximale du protecteur respiratoire (200) ; un clapet de valve (206) agencé au niveau de la première ouverture (201) pour s'engager avec faculté de fermeture avec un siège de vanne (207) circonférentiellement à la première ouverture (201), ledit clapet de valve (206) et ledit siège de vanne (207) créant une vanne principale qui est dans une position ouverte pendant une inspiration et dans une position fermée pendant une expiration végétative normale par le biais du protecteur respiratoire (200) ; un organe de ressort (208), le ressort sollicitant la partie distale (101, 201) relativement à la partie proximale (202), pour créer une vanne de détente, qui est dans une position fermée pendant une expiration végétative normale et passant dans une position ouverte lorsque la pression dans la trachée et ainsi dans le protecteur respiratoire (200) excède une valeur seuil correspondant à une haute pression inconfortable dans la trachée ;
dans lequel la partie distale (201) comprend un couvercle (211), ledit couvercle (211) comprenant la première ouverture (203) et pouvant tourner dans le plan transversal du protecteur respiratoire (200) relativement au clapet de valve (206), de telle sorte qu'un passage d'expiration libre est obtenu à travers la première ouverture (203) dans une position d'expiration libre.

2. Protecteur respiratoire selon la revendication 1, dans lequel la partie distale (201) est suspendue relativement à la partie proximale (202) via l'organe de ressort (208) centralement au protecteur respiratoire (200).

3. Protecteur respiratoire selon la revendication 1 ou 2, dans lequel la partie proximale (202) comprend une partie proximale supérieure (209) montée sur une partie proximale inférieure (210), et un filtre d'échange d'humidité et de chaleur (205) est agencé entre la partie proximale supérieure (209) et la partie proximale inférieure (210).

4. Protecteur respiratoire (200) selon l'une quelconque des revendications précédentes, comprenant en outre un filtre d'échange d'humidité et de chaleur (205) reçu dans ledit logement (200).

5. Protecteur respiratoire (200) selon l'une quelconque des revendications précédentes, dans lequel le clapet de valve (206) est un disque de valve avec une ouverture (213), ladite ouverture étant superposée sur ladite première ouverture (201) dans ladite position d'expiration libre.

6. Protecteur respiratoire (200) selon l'une quelconque des revendications précédentes, dans lequel le clapet de valve (206) est agencé sur une structure de support de partie distale (212) ayant au moins un piquage de verrouillage (214) s'étendant proximalement depuis ladite structure de support (212) pour coulisser avec faculté d'engagement dans la direction proximo-distale dans une rainure de verrouillage (215) agencée dans la direction proximo-distale sur la partie proximale (202), pour empêcher une rotation du clapet de valve (206) dans le plan transversal du protecteur respiratoire (200).

7. Protecteur respiratoire (200) selon la revendication 6, dans lequel le piquage de verrouillage (214) et la rainure de verrouillage (215) sont suspendus centralement au protecteur respiratoire (200).

8. Protecteur respiratoire (200) selon l'une quelconque des revendications précédentes, dans lequel la partie distale (201) comprend plusieurs premières ouvertures (203).

9. Protecteur respiratoire (200) selon la revendication 8, dans lequel les plusieurs premières ouvertures (203) sont distribuées de façon égale sur la surface d'extrémité distale de la partie distale (201).

10. Protecteur respiratoire (200) selon la revendication 9, dans lequel le clapet de valve (206) est un disque de vanne avec plusieurs ouvertures (213), lesdites ouvertures étant superposées sur lesdites plusieurs premières ouvertures (201) dans ladite position d'expiration libre.
